# EUROPEAN PATENT APPLICATION

(11) **EP 3 449 884 A1**
(43) Date of publication of application: **06.03.2019**
(21) Application number: 17189131.0
(22) Date of filing: 01.09.2017
(51) Int. Cl.: A61F 13/56

(54) **IMPROVEMENTS TO FASTENING SYSTEM OF DISPOSABLE ABSORBENT ARTICLES**

(71) Applicant: Grupo P.I. Mabe S.A. de C.V., Colonia Loma, Puebla 72230 (MX)
(72) Inventor: Canales Espinosa de los Monteros, Carlos, 72813 San Andrés Cholula, Puebla (MX); Zamudio Ahumada, Andrés, 72760 San Pedro Cholula, Puebla (MX)
(74) Representative: Macchetta, Andrea

(57) **Abstract**

The present invention protects, a disposable diaper with two opposite transverse sides, two opposite longitudinal sides, a front portion, a back portion and a crotch portion, basically formed by a chassis, a pair of front ears placed in the front area of the diaper, a pair of rear ears placed in the back area of the diaper, a traditional fastening system consisting of a pair of fastening elements joined or associated to the rear ears and a front band or tape and an additional fastening system, such that the additional fastening system is formed by at least one additional fastening element placed on the outer side of each one of the front ears of the diaper that are attached to the inner part of the diaper when placing it on the user.

## Description

### TECHNICAL FIELD

The invention pertains to the technical field of absorbent hygiene products. In particular, the present invention relates to a disposable diaper used to contain and retain bodily exudates and which is basically formed of an upper fluid-permeable layer, a lower fluid-impermeable layer and an absorbent core positioned between them, in addition to a fastening system to keep it in its use configuration when you place it on the user; they may also have anti-leakage barriers, one or more transfer or distribution layer or layers that give integrity to the absorbent core, and elastic areas in the crotch and waist regions or in any other region of the diaper.

### BACKGROUND

Nowadays, disposable diapers for babies and disposable diaper for adults with different configurations can be found in the market, one of these configurations that is generally accepted and desired by consumers, consists of a rectangular chassis with front and rear ears or panels on each side of the front and back portions of the chassis, in order to give an anatomical shape to the diaper; the chassis may or may not have a curved cut in the crotch to make it more comfortable for the user.

The fastening system of a disposable diaper is usually comprised by a pair of fastening tapes attached to the back waist region of the diaper, and a frontal tape or band placed on the external front waist region of it, sometimes called the "landing zone"; when the diaper is placed on the user, the fastening tapes are fastened on the frontal tape, so that the transversal edges of the back portion of the diaper, overlap on the transversal edges of the front portion of said diaper. The fastening system may have elastic zones, in order to achieve a better fit of the diaper to the user and it may use adhesive or mechanical fastening tapes in order to fasten on the frontal tape or band.

During use, with the user's movements, the overlapped ends of the front portion of the diaper, specifically the ears or frontal panels of the diaper, tend to move up or down, causing discomfort and increasing the chances of leakage due to disarrangement; on the other hand, with the advances in technology, the absorbent core of this type of articles can withstand several discharges of urine without leakage and keeping the user's skin dry, however, after two or three discharges of body fluids, the weight of the absorbent core of the diaper increases considerably and tends to pull down the diaper, which results in the overlapped zones of the front portion, previously placed underneath the lateral edges of the rear region of said diaper, to hang down causing misalignments, discomfort and, in extreme cases, leakages.

There are some proposals to improve fastening systems, which have, in addition to the traditional fastening system comprised by the fastening tapes and the frontal tape, a second fastening system; both systems work together to achieve a good fit in the diaper when placing it on the user and during the use of the article.

Some examples of these improved fastening systems are mentioned below:
The P&G patent US4699622, refers to a diaper that has a dual fastening system, a traditional system such as the one described above and an internal fastening system, placed on the outer layer of the diaper in its front zone, in the overlapping area, so that it connects the front and rear zones in said overlapping area, however the system described in US4699622A can weaken the outer layer because it is placed directly over it, in addition it complicates considerably the manufacturing process of the disposable diaper.

The Kimberly Clark patent US7569042 protects a diaper with dual fastening system, the first one formed by a first pair of fastening elements joined in the longitudinal ends of the rear waist region of the diaper and the second one by a second pair of fastening elements joined adjacent to the longitudinal ends of the front waist region of the diaper, so that the length of the second fastening elements is greater than the length of the first fastening elements and, in the case of a diaper with front and rear ears, the length of the front ear is greater than the length of the rear ear. When placing the diaper described in US7569042 on the user and overlapping the rear waist region on the front waist region, the front ears, which are longer than the rear ears are not fully covered by the rear waist region and the diaper does not take the desired shape, similar to cloth panties, in this type of articles.

Some other more recent proposals, describe mechanical fastening systems with a first fastening system consisting in a couple of fastening tapes with hooks and a frontal tape formed by loops ("velcro" system or Hook & Loop system) and a second fastening system associated to the frontal tape; this second fastening system consists of a pair of fastening elements formed by hooks, so when placing the diaper, these hooks are attached to the inner layer of the diaper usually made of a non-woven fabric having fibers in the form of loops on its surface so that these loops are joined with the hooks of the second fastening system. This is the case of the patent US6945968B2 and of the applications PCT WO2015015350 and WO2015015334A1. By associating the second fastening system to the frontal tape, the usefulness of the second fastening system is limited, since the two fastening systems, first and second, are very close to each other and, in the case of a diaper with front ears, these are not attached to any surface, so they are free to move up or down during use.

While the proposed fastening systems contribute to achieve a better fit of the diaper on the user and to maintain this adjustment during use, neither is designed to effectively eliminate the problem of misalignment of the ears or front panels during the use of the diaper. The present invention proposes a fastening system formed by a traditional fastening system and an additional fastening system designed to keep the front ears or panels attached to the inner part of the diaper to avoid any misalignment of the same. On the other hand, the proposed fastening system, contributes to further improve the processability of the production line.

The disposable absorbent article of the present invention may basically be described as being formed by a chassis, a pair of front ears and a couple of rear ears, the rear ears being longer and preferably wider than the front ears and preferably made of an elastic material, while the front ears are preferably rigid. The diaper also has an improved fastening system comprising a traditional fastening system and an additional fastening system. The traditional fastening system comprises, for example, a pair of fastening tapes and a frontal tape; the fastening tapes may be attached to or associated with the rear ears and when placing the diaper on the user said fastening tapes join to the frontal tape, which is placed in the front waist region on the outer portion of the diaper. The additional fastening system comprises, for example, a pair of fastening elements placed on the outer region of the frontal ears of the diaper, so when placing the diaper on the user these fastening elements may be attached to the inner part of the diaper, avoiding the front ears to slide up or down during use.

### OBJECTIVES OF THE INVENTION

An objective of the present invention is to use, in a disposable absorbent article, such as a diaper, an improved fastening system that prevents the front ears from sliding up or down during the use of the it.

A further objective of the invention is that the two fastening systems that form the improved fastening system of the invention, cooperate with each other to achieve a good fit and prevent the diaper from hanging because of the weight of the urine after multiple discharges.

An additional objective of the present invention is to improve the manufacturing process of the disposable diaper, avoiding waste and increasing productivity.

### SUMMARY OF THE INVENTION

The present invention provides a disposable absorbent article comprising two opposite transverse sides (4a,4b) running parallel to a transverse axis; two opposite longitudinal sides (3a,3b) extending between, and perpendicular to, said transverse sides (4a,4b); a front portion (5), a back portion (7), and a crotch portion (6) extending therebetween, forming a chassis (1) having a garment facing surface extending opposite a skin facing surface; a pair of front ears (15) coupled to the front portion (5) of the chassis (1) and having a garment facing surface extending opposite a skin facing surface; a pair of rear ears (14) coupled to the back portion (7) of the chassis (1) and having a garment facing surface extending opposite a skin facing surface; a first fastening system comprising one or more fastening elements (22) coupled to each of the rear ears (14), and a distinct frontal landing zone (24) for fastening of the one or more fastening elements (22) thereto; wherein the absorbent article comprises an additional fastening system, characterized in that the additional fastening system comprises, preferably consists of, at least one additional fastening element (23) positioned on the garment facing surface of each one of said front ears (15) and arranged such that the additional fastening element (23) is secured to the skin facing surface of the chassis and/or of the rear ears (14) when the absorbent article is worn by a user, and wherein the front ears (15) are shorter than the rear ears (14) along an axis perpendicular to the transverse axis (i.e. I < L) so that when worn by the user, said front ears (15) are completely covered by the back portion (7) of the chassis and/or rear ears (14), and wherein the shear force between the additional fastening element (23) and the skin facing surface of the chassis and/or of the rear ears (14), measured according the test method described in the description section, is at least 58 N/m, preferably between 58 and 386 N/m, more preferably between 60 and 380 N/m. It may additionally be advantageous that the front ears (15) are shorter than the rear ears (14) along an axis parallel to the transverse axis (i.e. a < A).

According to an embodiment, the additional fastening element (23) comprises, preferably consists of, one or more strips of hooks, disposed along each of the front ears. Advantageously, the additional fastening element (23) comprises, preferably consists of, a plurality of said strips.

According to another embodiment of the invention, the disposable absorbent article is characterized in that the additional fastening element (23) comprises, preferably consists of, one or more distinct patches comprising a plurality of hooks, said patches preferably having a shape selected from the group consisting of circles, squares, rectangles, triangles, hearts, stars, lines, pentagons, hexagons, octagons, trapezes, polygons and combinations thereof.

In these previous embodiments, the additional fastening element (23) may be disposed on the garment facing surface of each of the front ears and extend either along an axis perpendicular to the transversal axis, or along an axis parallel to the transversal axis. Preferably, it extends along an axis perpendicular to the transversal axis. It may advantageously comprise, preferably consist of, two or more parallel strips disposed on the garment facing surface of the front ears and extending along an axis perpendicular to the transversal axis. In an embodiment, the additional fastening element (23) covers at least 25%, preferably at least 40%, more preferably at least 50%, even more preferably at least 80%, most preferably from 90% to 100%, of the garment facing surface of each one of the front ears (15).

According to embodiments of the invention, the additional fastening element (23) is placed at a distance from a fixed edge (20) of the front ears (15), said distance being at least 25%, preferably at least 50%, more preferably at least 75%, even more preferably from 76% to less than 100%, most preferably from 80% to 90%, of an ear width "a".

In an embodiment, the front ears (15) comprise, preferably consist of, a non-woven fabric, preferably having a basis weight of less than 30 g/m², more preferably at most 25 g/m², or at most 20 g/m², most preferably at most 15 g/m². Preferably, said non-woven fabric has a basis weight of at least 5g/m², more preferably at least 10 g/m².

In an embodiment, the additional fastening element (23) comprise a plurality of distinct regions consisting of hooks and each separated by one or more further regions free of hooks, and wherein said plurality of distinct regions are aligned at least along an axis perpendicular to the transverse axis. Preferably, the plurality of distinct regions are skewed along an axis substantially parallel to the transverse axis, preferably such that an axis running parallel to the transverse axis and crossing the mid-point of one of said regions does not intersect with at least one, preferably all, neighboring distinct regions.

Preferably the fastening elements (22) are disposed on a skin facing surface of the rear ears (14).

Preferably the rear ears (14) are elastic and comprise an elastic web material, preferably comprising an elastic film laminated between at least two nonwoven layers. In that case, it may be advantageous that the elastic film between the at least two nonwoven layers be perforated to create corrugations into the nonwoven layer, thereby forming a plurality of peaks and valleys onto which hooks may adhere.

In an embodiment, a layer forming the skin facing surface of the chassis (1) and/or of the rear ears (14), to which the additional fastening element (23) is directly adhered to when the absorbent article is worn by a user, consists of a nonwoven layer, preferably a spunbond nonwoven layer. In that case, the nonwoven layer may be embossed or corrugated such that a plurality of peaks and valleys are formed onto which hooks may adhere.

### DESCRIPTION OF FIGURES

Figure 1 illustrates a disposable diaper according to the invention in extended form, showing its inner surface.
Figure 2 shows the disposable diaper of figure 1 in extended form, showing its outer surface.
Figures 3a and 3b show two configurations that could take a diaper according to the invention when is placed on user.
Figures 4a to 4h illustrate different configurations of the additional fastening element of the invention.
Figures 5a and 5b illustrate how the front and rear ears must be cut and placed on the Stainless Steel Plate for testing the shear force required to detach the additional fastening system of the invention.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or openended terms that specifies the presence of what follows e.g. component and do not exclude or preclude the presence of additional, non-recited components, features, element, members, steps, known in the art or disclosed therein.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, and the like. Absorbent articles preferably comprise a longitudinal axis and a transversal axis perpendicular to said longitudinal axis. The longitudinal axis is hereby conventionally chosen in the front-to-back direction of the article when referring to the article being worn.

As used herein, the "skin-facing" or "bodyside" surface means that surface of the article or component which is intended to be disposed toward or placed adjacent to the body of the wearer during ordinary use, while the "outward", "outward-facing" or "garment-facing" surface is on the opposite side, and is intended to be disposed to face away from the wearer's body during ordinary use. Such outward surface may be arranged to face toward or placed adjacent to the wearer's undergarments when the absorbent article is worn.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

The term "spunbond" refers to a substrate or layer having fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The present invention protects a disposable absorbent article, e.g. a diaper, with an improved fastening system. A disposable diaper according to the invention is preferably formed by a chassis, a pair of front ears, a pair of rear ears and the improved fastening system of the invention; the chassis preferably consists of an upper fluid-permeable layer, a lower fluid-impermeable layer and an absorbent core placed between these. The diaper preferably has a pair of longitudinal edges and a pair of transversal edges (one in the front and the second in the back portion) and is divided in three different portions, a front portion, a back portion and a crotch portion. The chassis may also have elastic zones, anti-leakage barriers, transfer and/or acquisition and distribution layers and other elements known in the art. The front ears are advantageously attached to the longitudinal edges of the chassis on the front portion, and the rear ears are advantageously attached to the rear longitudinal edges of the chassis, on the back portion.

One of the functions of the front ears of a diaper described in the invention, is to achieve a greater coverage of the user's waist when placing the diaper for its use, and are used generally in diapers which have a straight chassis. Considering the above, the front ears generally are wide enough to achieve this coverage and may have a shorter length than the length of the rear ears so that when overlapping the rear area on the front area of the diaper for placing the diaper on the user, the entire surface of the front ears is hidden in the overlapped portion, thus achieving an aesthetic appearance. To form the front ears of a disposable diaper, it is generally used a non-woven fabric with the softness and flexibility required to be in direct contact with the skin; on the other hand, considering that it is a consumer product that is discarded after use, the cost of the materials used must always be considered, so, it is not necessary that the material of the front ears have elasticity; besides, it should have the minimum basis weight (g/m²), so that it can be processed in high-speed production lines, and have the appropriate features so that when placing the diaper on the user, the front ears can be taken by the person who is placing the diaper without breaks or deformations; in addition it should be soft and flexible, since it will be in direct contact with the skin of the user during the use of the diaper.

The rear ears of a disposable diaper, in addition to cooperating with the coverage of the diaper at the waist, generally have the role to help achieve a good fit when placing it on the user; these rear ears are generally overlapping the front waist area of the diaper, so it may be convenient that they have elongation properties, as well as being wider and longer than the front ears, so that when placing the diaper on the user, said rear ears entirely cover the front ears.

The improved fastening system of the invention has, in addition to a traditional fastening system, an additional fastening system; the traditional fastening system preferably comprises, or consists of, one or more fastening elements fixed or connected to each of the rear ears of the article designed to attach to the front area of the chassis when placing the diaper on the user, either directly on the outer layer or on a frontal tape placed on the outer layer in the front portion of the chassis; the additional fastening system preferably comprises, or consists of, one or more fastening elements attached to each one of the front ears of the diaper, on the outer surface of the same, so that when placing the diaper on the user, these are attached to the inner layer of the diaper or to the inner surface of the rear ears, in the overlapping area.

These additional fastening elements may prevent the front ear from moving from its original position during the use of the product, causing misalignments of the diaper, discomfort to the user and potential leakage.

On the other hand, by using the additional fastening elements of the present invention, it is possible to reduce the basis weight of the layer that is used to form the front ears, thus obtaining a more profitable product. This is possible because the material of which the front ears are made, usually non-woven fabric, must have a certain basis weight in order to be processed in a production line of disposable diapers; by placing the additional fastening element in the front ears, it is possible to use a material with a less basis weight, since the additional fastening element increases the total weight of the ear and prevents it from flapping during the process; as well as when bending the diaper for packaging, it may avoid the front ears from wrinkling or folding onto themselves, which could cause problems when placing the diaper and could hurt the skin of the user. It is generally used a non-woven fabric with a minimum basis weight of 30 g/m² to form the front ears of a disposable diaper; when placing the additional fastening elements of this invention on said front ears, the weight of the non-woven fabric used to form them can be reduced up to 15 g/m², without sacrificing productivity, aspect or functionality of the final product.

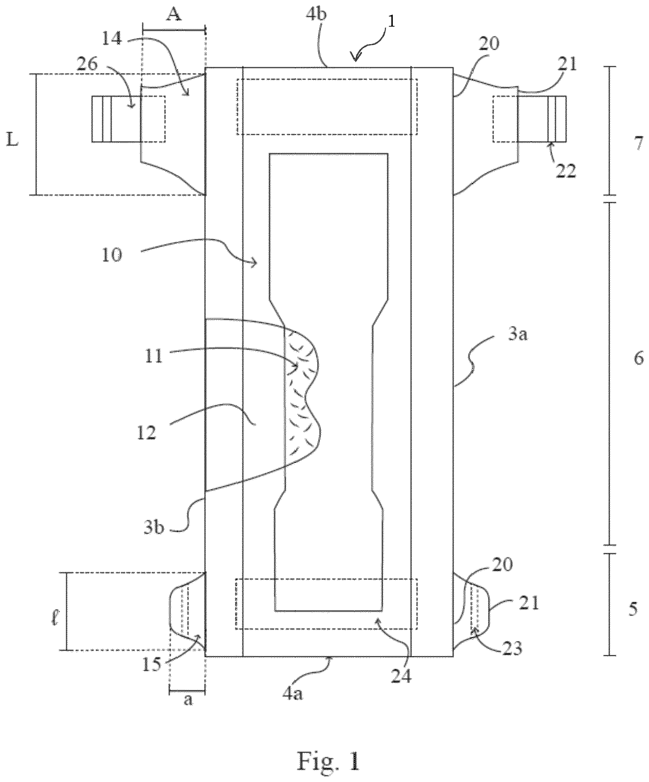

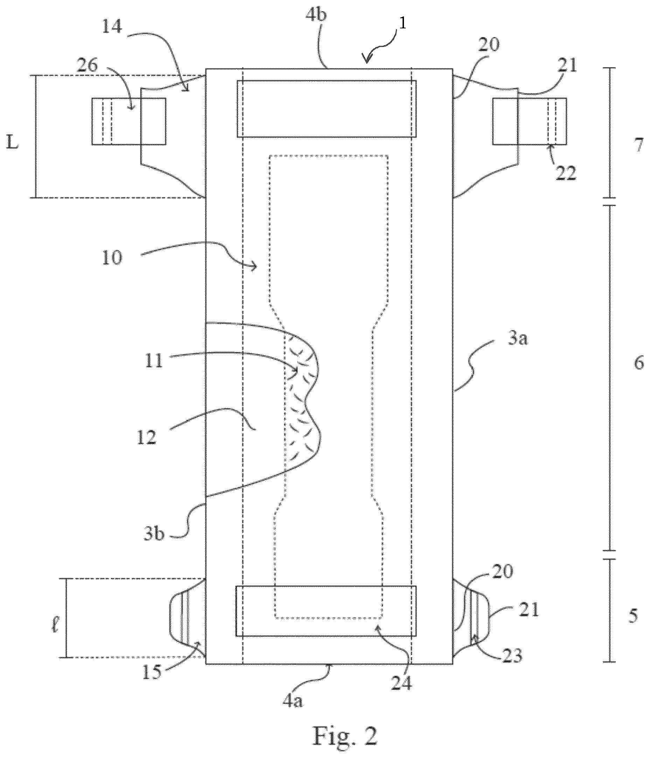

Figures 1 and 2 show a disposable diaper according to the invention in extended form, Fig. 1 shows the inner side and Fig. 2 shows the outer side of the diaper. As can be seen in this example, the diaper is formed by a chassis (1), a pair of front ears (15) a pair of rear ears (14) and an improved fastening system according to the invention. The chassis (1) has two opposite longitudinal sides (3a, 3b) and two opposite transverse sides (4a and 4b), which correspond to the front (4b) and rear (4a) edges of the diaper. The two opposite transverse sides (4a,4b) run parallel to a transverse axis, and the two opposite longitudinal sides (3a,3b) extend between, and perpendicular to, said transverse sides (4a,4b).

The diaper is divided in three portions, a front portion (5), a back portion (7) and a crotch portion (6) extending therebetween.

The chassis (1) has an upper fluid-permeable layer (10), a lower fluid-impermeable layer (12) and an absorbent core (11) placed between them; it may also have external anti-leakage barriers, one or more transfer, acquisition and/or distribution layers placed between the upper layer (10) and the absorbent core (11) and/or between the absorbent core (11) and the lower layer (9) (not shown in the figure), as well as elastic zones in waist and crotch portion (not shown in the figure).

The upper fluid-permeable layer (10) is oriented toward the skin of the user. Its main features are softness and permeability, it may consist of one or more layers of non-woven fabric of any suitable composition, and it may contain synthetic and/or natural fibers, bi-component fibers, multicomponent or nanofiber and can be, a spunbond, meltblown, carded, or any kind of nonwoven. In addition it can be smooth, embossed or perforated and have a design with patterns, images, colors, shapes, drawings, or their combinations.

The lower fluid-impermeable layer (12) is oriented toward the garment of the user, it retains the corporal fluids and prevents the garment(s) in contact with the diaper from getting dirty. Can be used as outer layer any type of waterproof film with the appropriate properties of softness, flexibility, stretching, resistance, caliber, weight and cost to be used in a disposable diaper; can be made up of natural and/or synthetic compounds, such as polyethylene, polypropylene, polyester, polystyrene, polylactic acid, etc. ; this film may be of any color or be printed with any type of pattern and by any means; in addition, it can be laminated to a layer of non-woven fabric, so that the outer part of the chassis has a cloth appearance; could be used any type of non-woven fabric suitable for the lamination process (which can be done by any means known) and with the right features of softness, flexibility, cost and tensile stress, to be used in a disposable diaper; it can be smooth, embossed or perforated and have a design with patterns, images, colors, shapes or drawings that will determine the outside appearance of the absorbent article.

The absorbent core (11) which is placed between the upper fluid-permeable layer (10) and the lower fluid-impermeable layer (12), has the function of absorption and retention of fluids and/or bodily exudates. It is usually made up of absorbent material like cellulose fibers and/or of particles or fibers of super absorbent material or of any type of material suitable for this purpose; it can take any form that helps to achieve a good fit of the diaper on the user.

Elastic crotch region (not shown in figures) are mainly arranged along the longitudinal sides of crotch portion (6) of the diaper and may be composed of any type of elastic or elastomeric material attached to the chassis (on or between the layers that form it), in a tensed way. The elastic or elastomeric elements can be threads, bands or elastic or elastomeric laminates.

The anti-leakage barriers (not shown in figures) have the function of avoiding side leakage of the liquid, solid or semi-solid excretions; they are formed, preferably of hydrophobic non-woven fabric and are positioned inside each longitudinal side (3a,3b) of the chassis (2), have a proximal end (which is attached to the inner layer (10) and/or to the outer layer (12) in the region adjacent the absorbent core (11)), and a distal end away from the absorbent core by means of any elastic means.

The front (15) and rear (14) ears of a disposable diaper according to the invention, preferably have an edge attached to the chassis or fixed edge (20) and a distal edge (21), a maximum length "l" for the front ears and "L" for the rear ears, that corresponds to the length of the fixed edge (20) and a maximum width "a" for the front ears and "A" for the rear ears, that corresponds to the maximum transverse distance from the fixed edge (20) to the distal edge (21). As mentioned, the role of the front ears (15) is to achieve greater coverage in the front waist area when placing the diaper on the user, while the rear ears (14) have an important role in the adjustment of the diaper on the user. For this reason, the front ears (15) are preferably narrower and/or shorter than the rear ears (14), so that when placing the diaper on the user, the front ears (15) are preferably completely covered by the overlapping area, resulting in a diaper that takes a very similar shape to a cloth diaper. Considering the above, for the purposes of the invention, l<L and preferably a<A.

The fastening system of the invention comprises a first, herein sometimes called "traditional", fastening system and an additional fastening system. The traditional fastening system comprises one or more, preferably a pair of, fastening elements (22), coupled or attached directly or indirectly to each of the rear ears (14), advantageously to the distal edge (21) of each of the rear ears (14) of the diaper and a frontal landing zone(24), sometimes herein called "tape or band", generally placed on the outer side of the diaper at its front waist portion, on which the fastening element (22) will attach when placing the diaper on the user.

Figure 1 illustrates a traditional fastening system in which, the fastening element (22) is attached to a fastening tape (26), which is joined to the distal edge (21) of each of the rear ears (14), however the fastening element (22) can be attached directly to the distal edge (21) of each of the rear ears (14) or on each of the rear ears (14).

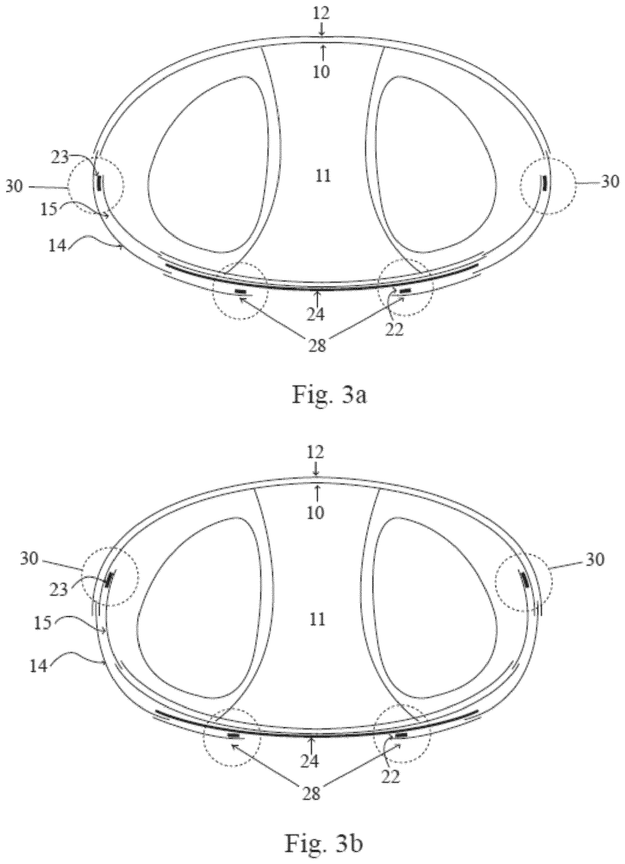

The additional fastening element (23) is placed on the outside of each of the front ears (15), so that when placing the diaper on the user it joins the inner side of the back portion (7) which overlaps the front portion in the user configuration of the diaper, either to the body facing side of the rear ears (14) or directly to the inner layer (10) of the diaper, creating, in conjunction with the traditional fastening system, four joining areas between the front (5) and back (7) portions of the diaper: as can be seen in Figures 3a and 3b, two front joining areas (28) and two lateral joining areas (30), that cooperate between them to hold the diaper in place and prevent that during use, the front ears (15) slide in and out through the front waist edge of the diaper or through its crotch area as a result of the movements of the user or the weight of the absorbent core (11).

The two front joining areas (28) are formed by joining the fastening elements (22) with the frontal tape (24) and the two lateral joining areas (30), are formed by the union of the additional fastening elements (23) to the body facing side of the rear ears (14) or to the inner layer (10) of the diaper. Figures 3a and 3b, illustrate the two alternatives described, in the alternative shown in figure 3a, the additional fastening elements (23) are attached to the inner layer of the diaper, while in the alternative shown in figure 3b, the additional fastening elements (23), are attached to the inner part of the rear ears (14).

For the purposes of the present invention, the closer the front joining areas (28) with the lateral joining areas (30), the lesser the effectiveness of the improved fastening system, because this increases the risk of the front ears misalignment; that is why the additional fastening element (23) is preferably positioned at a distance from the fixed edge (20) of each of the front ears (15) of at least 25% of the maximum width "a" of the front ear (15) and preferably of at least 50% of the maximum width "a" of each one of the front ears (15) and more preferably at a distance of 75% of the maximum width "a" of each one of the front ears.

The lateral joining areas (30) generally prevent the front ears (15) from sliding up or down during the use of the diaper and cooperate with the traditional fastening system to keep diaper from hanging down during use, causing misalignment, discomfort, and runoffs.

The fastening elements (22,23) used in both systems, traditional and additional, are preferably mechanical, although can also be used adhesive fastening elements or a combination of both, in other words, the traditional fastening elements (22) could be adhesive, while the additional fastening elements (23) could be mechanical or vice versa.

As additional fastening element (23) is generally used one or more strips of hooks , preferably of at least 2 mm wide placed parallel to the longitudinal axis of the article and disposed along each of the front ears; the additional fastening element (23) may be joined by adhesive, ultrasound or other suitable way, along each of the front ears and at a specific distance from its fixed edge (20). In a modality of the invention, the one or more strips of hooks are skewed along an axis substantially parallel to the longitudinal axis.

To measure the bond strength of the fastening systems for disposable diapers, there are usually two types of measurements: shear force and peel force The first one is used to determine the strength required so that the fastening elements detach by themselves because of the forces applied during use and the second one to determine the strength required in order for the caregiver to detach the tapes to re-locate or to change the diaper.

In the case of a traditional fastening system, it has been determined that the shear force required to detach the fastening element (22) from the frontal tape (24) is usually at least 386 N/m. With the benefit of the present invention, it may be reduced to a shear force of less than 386 N/m, preferably less than 380 N/m.

Considering that the additional fastening element (23) of the present invention does not work in the same way as the traditional fastening element since during use it is placed between two layers, and also because the forces applied on it are not cross-cutting as in the case of the traditional fastening element, a test method was designed to determine the shear force required so that the additional fastening element (23) detaches from the front ear during the use of the article. This results in a minimal force, measured according to the method described below, of 58 N/m, preferably between 58 and 386 N/m, more preferably between 60 and 380 N/m.

In an embodiment of the invention, the shear force required to detach the fastening element (22) from the frontal tape (24) is less than 386 N/m and the shear force between the additional fastening element (23) and the skin facing surface of the chassis and/or of the rear ears (14) is at least 58 N/m.

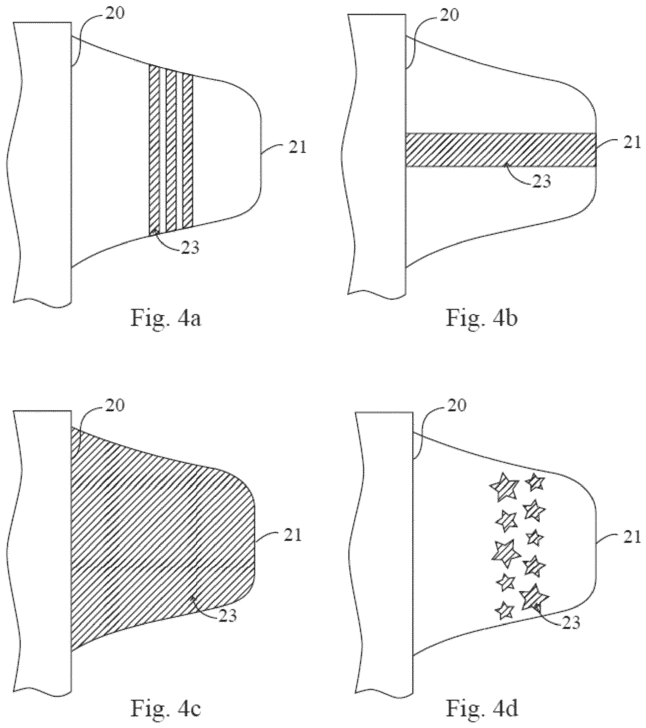

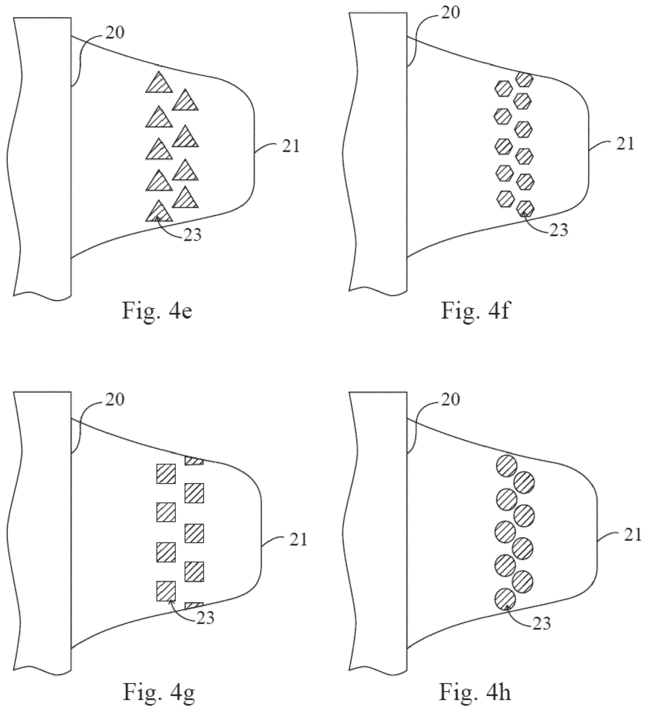

In an embodiment of the invention, the additional fastening element(s) (23) covers at least 25%, preferably at least 40%, more preferably at least 50%, even more preferably at least 80%, most preferably from 90% to 100%, of the garment facing surface of each one of the front ears (15), as shown in figure 4c.

Finally, the additional fastening element(s) (23) may have any appropriate settings for the purpose of the present invention, such as circles, squares, rectangles, triangles, hearts, stars, lines, pentagons, hexagons, octagons, trapezes, polygons or any other desired shape and may cover the entire length of each of the front ears, and/or may be shorter than these, as shown in Figures 4a to 4h.

### TEST METHOD: SHEAR FORCE FOR ADDITIONAL FASTENING ELEMENT

### Materials:

- Stainless Steel Plate 10.0 x 15.0 cm
- Double-sided adhesive tape
- Dynamometer

### Procedure:

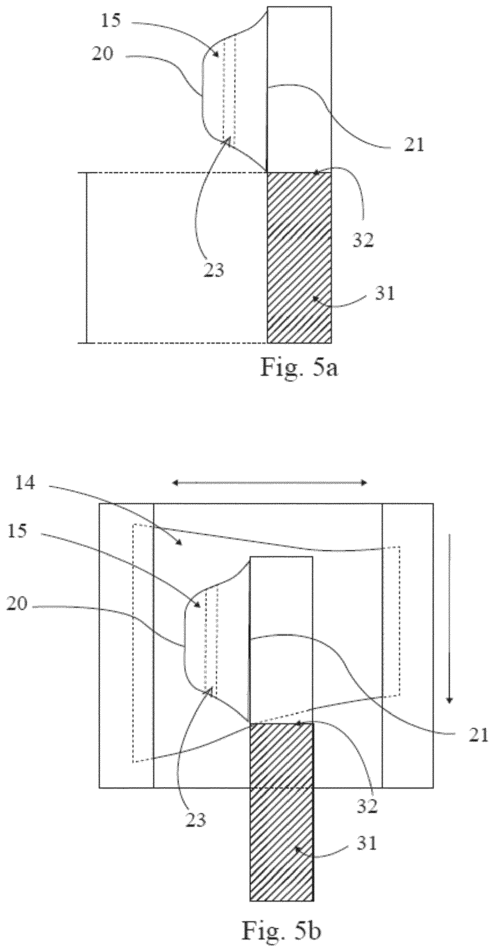

- Calibrate the dynamometer as follows:
   ∘ Separation of the clamps 120 mm
   ∘ Distance from the top edge of the steel plate to the edge of the upper clamp: 30 mm
   ∘ Speed: 12 in/min
- The diaper front ears (15), containing the additional fastening element (23), are cut from a diaper. Taking care of including the part of the front ear (15) attached to the chassis and a bottom strip (31) that extends from the diaper downwards from the attached edge (32) 100 mm (see figure 5a).
- Rear ears (14) are cut of the diaper.
- Both ears are held to the metal plate in the appropriate setting so that the additional fastening element (23) contacts the inner part of the rear ear (14), simulating the position it will take during the use of the article (see figure 5b).
- The additional fastening element (23) is bonded to the inner part of the rear ear (14).
- A weight of 2 kg is placed on the front ear (15), matching with the additional fastening element (23), during 2 minutes.
- The metal plate is placed in the back clamp of the dynamometer in such a way that the strip (31) is positioned upwards.
- The strip (31) is placed in the upper clamp (Movable clamp) of the dynamometer.
- The dynamometer is activated recording the force of detachment of the additional fastening element (23) along the attachment plane.

Although the invention submitted is described in detail in function of the preferred forms of implementation, it is not restricted to other forms of implementation and additional modifications that are within the spirit and scopes thereof, being evident that these are included within the same. In the same way claims are appended with the scopes that could present the invention.

## Claims

1. A disposable absorbent article comprising:
two opposite transverse sides (4a,4b) running parallel to a transverse axis;
two opposite longitudinal sides (3a,3b) extending between, and perpendicular to, said transverse sides (4a,4b);
a front portion (5), a back portion (7), and a crotch portion (6) extending therebetween, forming a chassis (1) having a garment facing surface extending opposite a skin facing surface;
a pair of front ears (15) coupled to the front portion (5) of the chassis (1) and having a garment facing surface extending opposite a skin facing surface;
a pair of rear ears (14) coupled to the back portion (7) of the chassis (1) and having a garment facing surface extending opposite a skin facing surface ;
a first fastening system comprising one or more fastening elements (22) coupled to each of the rear ears (14), and a distinct frontal landing zone (24) for fastening of the one or more fastening elements (22) thereto;
wherein the absorbent article comprises an additional fastening system, **characterized in that** the additional fastening system comprises, preferably consists of, at least one additional fastening element (23) positioned on the garment facing surface of each one of said front ears (15) and arranged such that the additional fastening element (23) is secured to the skin facing surface of the chassis and/or of the rear ears (14) when the absorbent article is worn by a user, and wherein the front ears (15) are shorter than the rear ears (14) along an axis perpendicular to the transverse axis so that when worn by the user, said front ears (15) are completely covered by the back portion (7) of the chassis and/or rear ears (14), and wherein the shear force between the additional fastening element (23) and the skin facing surface of the chassis and/or of the rear ears (14), measured according the test method described in the description section, is at least 58 N/m.

2. A disposable absorbent article according to Claim 1, **characterized in that** the additional fastening element (23) comprises, preferably consists of, one or more strips of hooks, disposed along each of the front ears.

3. A disposable absorbent article according to Claim 2, **characterized in that** the additional fastening element (23) comprises, preferably consists of, a plurality of said strips.

4. A disposable absorbent article according to Claim 1, **characterized in that** the additional fastening element (23) comprises, preferably consists of, one or more distinct patches comprising a plurality of hooks, said patches preferably having a shape selected from the group consisting of circles, squares, rectangles, triangles, hearts, stars, lines, pentagons, hexagons, octagons, trapezes, polygons and combinations thereof.

5. A disposable absorbent article according to any of the preceding Claims, **characterized in that** the additional fastening element (23) is disposed on the garment facing surface of each of the front ears and extends either along an axis perpendicular to the transversal axis, or along an axis parallel to the transversal axis.

6. A disposable absorbent article according to Claim 5, **characterized in that** the additional fastening element (23) comprises, preferably consists of, two or more parallel strips disposed on the garment facing surface of the front ears and extending along an axis perpendicular to the transversal axis.

7. A disposable absorbent article according to any of the preceding Claims, **characterized in that** the additional fastening element (23) covers at least 25%, preferably at least 40%, more preferably at least 50%, even more preferably at least 80%, most preferably from 90% to 100%, of the garment facing surface of each one of the front ears (15).

8. A disposable absorbent article according to any of the preceding Claims, **characterized in that** the additional fastening element (23) is placed at a distance from a fixed edge (20) of the front ears (15), said distance being at least 25%, preferably at least 50%, more preferably at least 75%, even more preferably from 76% to less than 100%, most preferably from 80% to 90%, of an ear width "a".

9. A disposable absorbent article according to any of the previous claims, **characterized in that** the front ears (15) comprise, preferably consist of, a non-woven fabric, preferably having a basis weight of less than 30 g/m², more preferably at most 25 g/m² or at most 20 g/m², most preferably at most 15 g/m².

10. A disposable absorbent article according to any of the preceding Claims **characterized in that** the additional fastening element (23) comprise a plurality of distinct regions consisting of hooks and each separated by one or more further regions free of hooks, and wherein said plurality of distinct regions are aligned at least along an axis perpendicular to the transverse axis.

11. A disposable absorbent article according to Claim 10 **characterized in that** the plurality of distinct regions are skewed along an axis substantially parallel to the transverse axis, preferably such that an axis running parallel to the transverse axis and crossing the mid-point of one of said regions does not intersect with at least one, preferably all, neighboring distinct regions.

12. A disposable absorbent article according to any of the preceding Claims **characterized in that** the fastening elements (22) are disposed on a skin facing surface of the rear ears (14).

13. A disposable absorbent article according to any of the preceding Claims **characterized in that** the rear ears (14) are elastic and comprise an elastic web material, preferably comprising an elastic film laminated between at least two nonwoven layers.

14. A disposable absorbent article according to any of the preceding Claims **characterized in that** a layer forming the skin facing surface of the chassis (1) and/or of the rear ears (14), to which the additional fastening element (23) is directly adhered to when the absorbent article is worn by a user, consists of a nonwoven layer, preferably a spunbond nonwoven layer.

15. A disposable absorbent article according to Claim 14 **characterized in that** the nonwoven layer is embossed or corrugated such that a plurality of peaks and valleys are formed onto which hooks may adhere.
